# EUROPEAN PATENT APPLICATION

(11) **EP 0 950 710 A2**
(43) Date of publication of application: **20.10.1999**
(21) Application number: 99106794.3
(22) Date of filing: 06.04.1999
(51) Int. Cl.: C12N 15/12, C07K 14/715, C07K 14/72, C12N 15/62, C07K 19/00, G01N 33/68

(54) **Cells expressing a chimera receptor and their uses**

(30) Priority: 03.04.1998 JP 10876998
(71) Applicant: JCR PHARMACEUTICALS CO., LTD., Ashiya, Hyogo 659-0021 (JP)
(72) Inventor: Takahashi, Ken-ichi, Tarumi-ku, Kobe, Hyogo (JP); Yagi, Toshihiro, Nagata-ku, Kobe, Hyogo (JP); Shirono, Hiroyuki, Tarumi-ku, Kobe, Hyogo (JP); Koga, Jun-ichi, Nishi-ku, Kobe, Hyogo (JP)
(74) Representative: Best, Michael, Dr.

(57) **Abstract**

The present invention provides a chimera gene as constructed by fusing the gene for the extracelluar region of human growth hormone receptor with the genes for the transmembrane and cytoplasmic regions of thrombopoietin receptor, an expression plasmid for the said gene and animal cells capable of expressing the protein encoded by the said gene.
The human-growth-hormone dependent animal cell line transformed with an expression plasmid for the said gene are useful in screening hormones which show affinity for the chimera receptor, as well as their agonists and antagonists.

## Description

The present invention relates to the construction of a chimera gene GHR/Mp1 by fusing the gene in the extracellular region of human growth hormone receptor with the genes in the transmembrane and cytoplasmic regions of the thrombopoietin receptor c-Mp1, to its expression plasmid and to animal cells capable of expressing the protein for the gene. Furthermore, the invention provides a method for constructing various receptors and is concerned with a method for functionally screening hormone compounds which show affinity for the chimera receptor as well as their agonists and antagonists.

Human growth hormone (hGH), a hormone secreted by the pituitary gland, is known to perform the body-growth promoting function either through production of somatomedins (IGF-1) in the liver or through direct action on the target tissue at the growth hormone receptor (GHR), and also to be involved in the metabolism of sugars and lipids, protein anabolism, and cell proliferation and differentiation at the tissue level [Wallis, M. et al., Elsevier Science Publishers BV 265, (1988)].

Currently, the titer assay for hGH includes the in vivo neck-bone apophysis cartilage amplification-increase test method (the tibia test) in pituitary-excised rats using as an index a weight-gain and cartilage-elongation, and the in vitro method for measuring Nb2-cell proliferative activity by taking advantage of crossreaction of hGH with prolactin receptor [Tanaka, T. et al., I. Clin. Endocrinol. Metab., 51: 1058 (1980)]. In addition, there may be mentioned the method for measuring the IM-9 receptor regulation activity [Rosenfeld R. G. et al., J. Clin. Endocrinol. Metab. 50: 62 (1980), Gavin III Jr. et al., J. Clin. Endocrinol. Metab., 55: 133 (1982)] which utilizes as an index phosphorylation of tyrosine, or a cytoplasmic signal transducing molecule upon hGH stimulation in IM-9 cells having GHR but not showing hGH-dependent growth and a method for measuring differentiation activity of 3T3-F422 fat cell line [Bristow A. F. et al., Pharmeuropa 3 (si): 3, (1991), Hayakawa T. et al., Pharmeuropa 3 (si): 33 (1991)].

Miscellaneous test methods include, for example, a radioreceptor assay (RRA) which comprises allowing GHR on the membrane fraction from IM-9 cells or liver cells of a pregnant house rabbit to compete with ¹²⁶I-labeled hGH and ELISA based on the use of an antibody specific to hGH [Nederman T. et. al., J. Biol. Stand 15: 199, (1987), Campino c., J. Clin. Endocrinol. Metab. 70: 601 (1990)].

The hGH receptor (GHR), belonging to the cytokine receptor superfamily, expresses in the liver and many other tissues [Leung D.W. et al., Nature 330: 537 (1987)]. It is widely known that when hGH, or a GHR-binding protein (ligand), binds to GHR, there is produced the GHR dimer, resulting in activation of lots of intracellular signal transducing molecules inclusive of non-receptor type tyrosine kinase to thereby bring about diverse physiologic effects.

GHR, in its extracellular region, possesses a unit which consists of a domain having four preserved cysteine residues and a domain comprising a characteristic sequence (WSXWS motif) of tryptophan-serine-X-tryptophan-serine, whereas it lacks a tyrosine-kinase like domain in its cytoplasmic region, and has the highly homologous regions, Box1 and Box2 [Koezuka, N., Igakuno Ayumi, 161: 74 (1992)].

On the other hand, thrombopoietin (TPO) is a cytokine which exhibits specific platelet-augmenting activity, as discovered in the form a ligand for the orphan receptors. [de-Sauvage, F. et al., Nature 369: 533 (1994), Kauhansky, K. et al., Nature 369: 568 (1994)].

Although a chimera-type antibody (Japanese Unexamined Patent Publication No. Hei-05/304982) and chimera plasmid (Japanese Unexamined Patent Publication No. Sho-60/58078) have been reported so far in the past, there has not yet been known the construction of chimera receptors for cytokines.

### [The Problem That the Invention Is Intended to Solve]

When in addition to the above various methods, the cell line and assaying method could be established to functionally analyze the actions of the hormones with their receptors at the cellular level, this would offer a novel technical means for investigating the physiologic effects of the hormones and screening useful substances. For example, preparation of a hGH-dependent cell line capable of proliferating only in the presence of hGH is expected to be of use in screening the agonist-like or antagonist-like active substances for hGH. Such hGH-dependent cells would desirably be detectable by means of cytosenser analysis in which a cell response to hGH is assayed in terms of changes in extracellular acidification ratio or rate as an index.

However, response to hGH varies greatly from cell to cell, while IM-9 cell does not demonstrate hGH-dependent extracellular proton release. And thrombopoietin (TPO)-dependent cell line (BaF/Mp1) proliferates not only owing to thrombopoietin but also through crosslinking of c-Mp1 membrane protein with anti-Mp1 monoclonal antibody, and gives rise to increases in extracellular acidification ratio according to the concentration of said monoclonal antibody.

The present inventors constructed a gene for the chimera receptor consisting of the extracellular region of GHR and the transmembrane and cytoplasmic regions of c-Mp1, the receptor for thrombopoietin (TPO), and succeeded in the establishment of the novel hGH-dependent cell line BaF/GM by introducing the gene into the mouse IL-3 dependent cell line BaF3.

The present invention relates to a chimera gene (GHR/Mp1) consisting of a gene coding for the extracellular region of human growth hormone receptor (hGHR) and a gene coding for the membrane spanning and cytoplasmic regions of thrombopoietin receptor (c-Mp1), as well as to a hGH-dependent cell line BaF/GM having such chimera gene introduced into a mouse IL-3 dependent cell BaF3 and to its uses.

The construction of the chimera gene may be exemplified as follows:

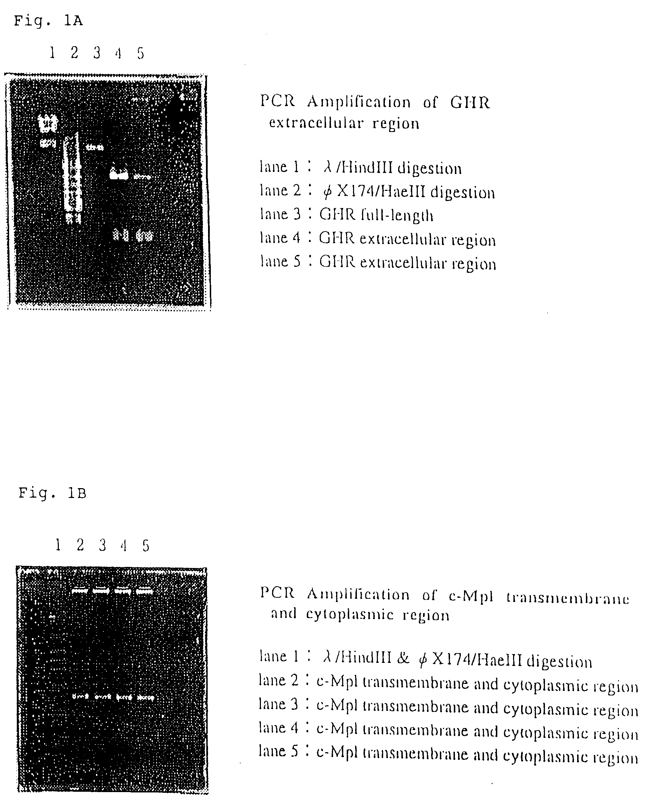

By using the RT-PCR technique with poly A⁺ RNA originated from the human liver, the extracellular region of human growth hormone receptor was amplified to synthesize cDNA with ca. 0.8 kbp (Fig. 1). By means of the RT-PCR technique with poly A⁺ RNA from the human fetal liver, the genes for thetransmembrane region and intraplasmic region of c-Mp1 were amplified to synthesize cDNA with ca. 0.7 kbp (Fig. 1B).

These cDNAs were inserted into an expression vector for animal cells pRC3-Uni (Fig. 2), followed by integration into TOP10F' competent cells of E. coli to generate transformants. Out of them, colony PCR was effected with 16 clones to select 8 clones (Fig. 3 A, B). Then, the selected clones were determined for the total nucleotide sequences, and selection was made of the clones CHR-EX and c-Mp1-TM+CP which are usable in the present invention. Plasmid GHR-Ex was double-digested with the restriction enzymes M1u1 and Mp1-CP to open the strands, into which the M1u1-Xho1 treated fragment of Mp1-CP encoding the transmembrane and cytoplasmic regions of Mp1 was inserted to thereby construct the recombinant plasmid containing the GHR/Mp1 receptor gene and this gene. (Fig. 4A, B).

Also, the preparation of a hGH-dependent cell line may be exemplified as follows:

Out of the chimera-receptor expression plasmid constructed, the clone pCR3-Uni/GM was selected and then inserted into E. coli to generate pCR3-uni/GM in large quantities. Furthermore, the generated clone was integrated into BaF3 cells by means of the DEAE-dextran method, and the resultant transformed cells were subjected to selection on a medium containing neomycin (G418), followed by additional selection on a medium containing hGH to establish the hGH-dependent cell line BaF/GM.

Construction of the GHR/Mp1 chimera receptor gene now enabled diverse dynamic analyses by means of cytosenser analysis to be performed thanks to its newly conferred capability to cause extracellular acidification in addition to proliferation in response to added hGH.

With reference to the signal transduction mechanism for GHR, the JAK2-STAT5 route and the Ras-Raf-MAPK route have been identified. Referring to the mechanism of bringing about the extracellular acidification of c-Mp1, it has been confirmed that formation of c-Mp1 dimer with anti-c-Mp1 monoclonal antibody leads to activation of various signal transduction molecules in the cell, thus causing extracellular acidification.

Increases in the extracellular proton concentrations are glucose-dependent, and this is assumed to be attributable to the fact that phosphatidylinositol-3 kinase (PI3K) exerts action as a signal transduction molecule on the glucose-dependent acidification to thereby generate phosphatidylinositol 3-phosphate (PI3P), which in turn gives rise to vesicular transport to allow the glucose transporter (GLUT) incorporated into the vesicles to express on the cell membranes.

The present invention encompasses the construction of the genes for chimera receptors consisting of the extracellular regions of various cytokine receptors and the transmembrane and cytoplasmic regions of c-Mp1 and their expression plasmids, as well as animal cells having such chimera genes expressed therein. As the cells which are utilizable as a host, the CHO cells, COS cells and others may be arbitrarily selected.

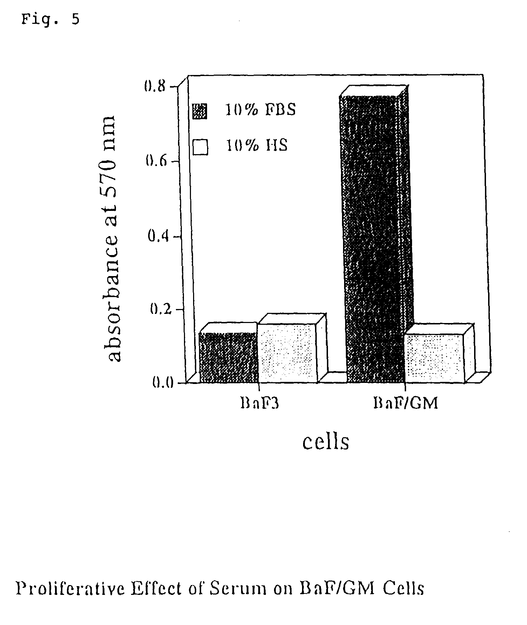

The BaF/GM cell of the present invention is able to proliferate in the presence of hGH or mIL-3 alone, and also shows potent growth on a medium containing 10% of fetal bovine serum (FBS), although it fails to grow on the fetal bovine serum medium containing 10 % of equine serum (HS), revealing that some factor contained in FBS exerts functional effects on the GHR/Mp1 chimera receptor expressed in BaF/GM cells. On the other hand, the BaF3 cell does not exhibit growth on a medium containing FBS and HS (Fig. 5). Proliferation of the BaF/GM cell after FBS supplementation is assumed to be attributed to the action of bovine growth hormone (bGH) contained in FBS.

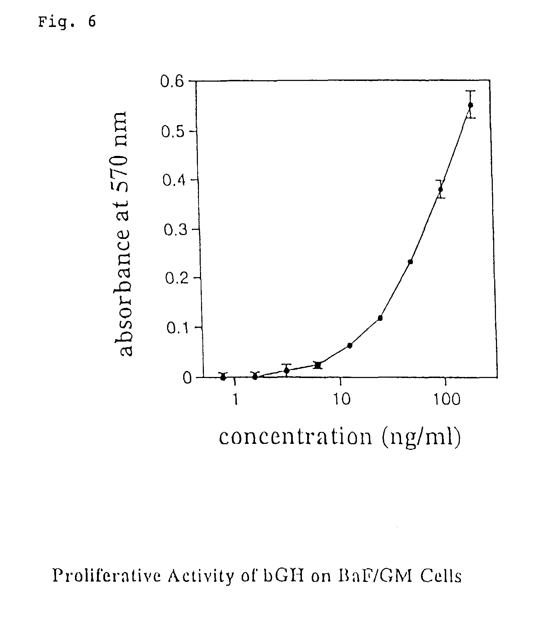

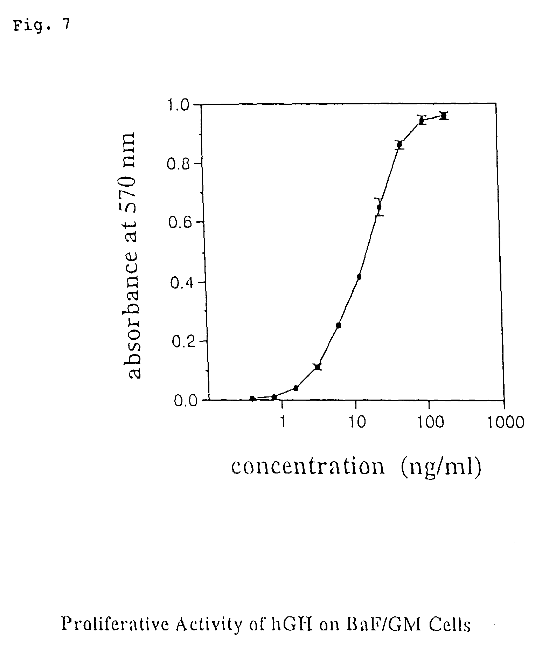

A comparative growth analysis for the BaF/GM cell indicates that the growth curve can be determined at concentrations in the range of 10 to 100 ng/ml for bGH and 1 to 200 ng/ml for hGH (Fig. 7), leading to the conclusion that bGH can exerts about 1/10 or so less the functional effect as than hGH (Fig. 6). As is evident from the above, the present invention is useful in the analysis of the species-specificity of hormones to cytokine receptors.

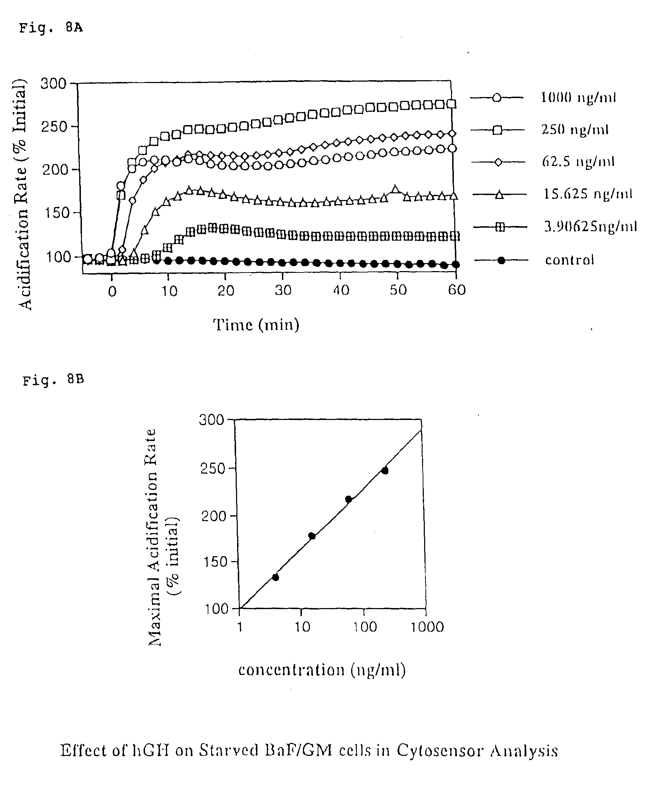

An analysis of responses of hGH to the BaF/GM cell reveals that rises in the concentration-dependent ratio or rate of extracellular acidification can be suppressed at concentrations ranging from 4 to 250 ng/ml, with saturation of such acidification ratio being noted at increased concentrations as high as 1 ug/ml (Fig. 8). This demonstrates that the chimera receptor expression cells will permit dynamic analysis of hormone actions.

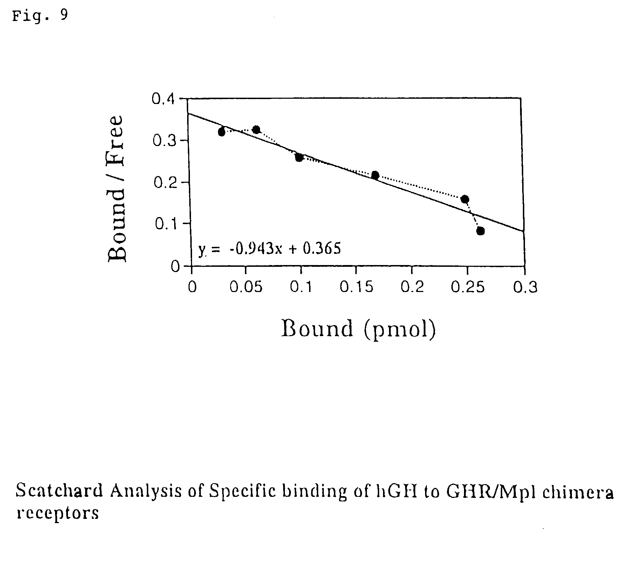

As is clarified by Scatchard analysis, the expressed number of the GHR/Mp1 chimera receptor in the BaF/GM cell and the dissociation constant of the chimera receptor to hGH were calculated at about 3,700 receptors per cell and 0.16 nM, respectively (Fig. 9). The calculated dissociation constant was in fair agreement with the dissociation constant (Kd = 0.17 to 0.19 nM) reported with human growth hormone binding protein (GHR extracellular domain) [Cunningham, B. C. et al., Science, 254: 821 (1991)]. These results manifest that the extracellular region of the chimera receptor is capable of binding like the naturally occurring hormone receptor, proving that the preparation of the chimera receptor is effective and useful in practice.

The BaF/GM cell line established according to the present invention, which possesses the GHR/Mp1 receptor exactly equivalent to GHR in terms of interaction with hGH and exhibits hGH-concentration-dependent cell growth and rises in extracellular acidification ratio, can therefore be used in screening the agonists or antagonists. In addition, the present invention can find practical application in establishing the chimera receptor genes for various cytokines and their expression plasmids as well as their expression cells.

Below described are the examples to illustrate the present invention in more detail, while referring to the drawings wherein:
Fig. 1A depicts an electrophoretic analysis of the PCR amplification products for the nucleotide sequence of the GHR extracellular region, as conducted in Example 1 (B). wherein; Lanes 1, 2, 3, 4 and 5 correspond to λ/HindIII digestion product, φX174/HaeIII digestion product, GHR full-length, GHR extracellular region and GHR extracellular region, respectively, and it is noted that the full-length and extracellular-region genes of GHR were amplified through PCR using cDNA from human liver, with the cleavage site of the restriction enzyme Mlul being incorporated into the used GHR extracellular-region gene on the 3'-end side during PCR amplification;
Fig. 1B depicts an electrophoretic analysis of the PCR products for the nucleotide sequences of the transmembrane and cytoplasmic regions of c-Mp1, as conducted in Example 1, wherein; Lane 1 corresponds to λ/HindIII & φX174/HaeIII double digestion, while Lanes 2 through 5 correspond to c-Mp1 transmembrane and cytoplasmic region, and it is noted that the transmembrane and cytoplasmic-region gene of c-Mp1 was amplified through PCR using cDNA from fetal human liver, with the cleavage site of the restriction enzyme Mlul being incorporated into the gene on the 5'-end side during PCR amplification;

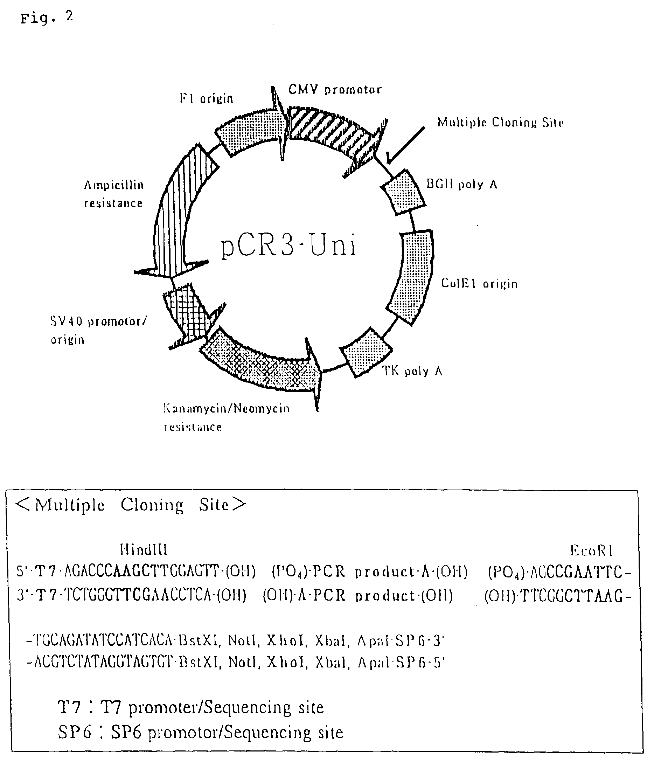

Fig. 2 illustrates the multiple cloning site in the genetic map of the plasmid pCR3-Uni;
Fig. 3A depicts an electrophoretic analysis for colony-PCR screening of GHR clones, as conducted in Example 1 (e), in which;
   Lane 1 corresponds to λ/HindIII & φX174/HincII double digestion,
   while Lanes 2 through 17 correspond individually to Clone #1 through Clone #16, and it is added that PCR was conducted with each clone lysate using Primer T7 and GR 4 to thereby select eight clones in total of Clones #2, #3, #4, #5, #6, #7, #8 and #9 with the given DNA sequence;

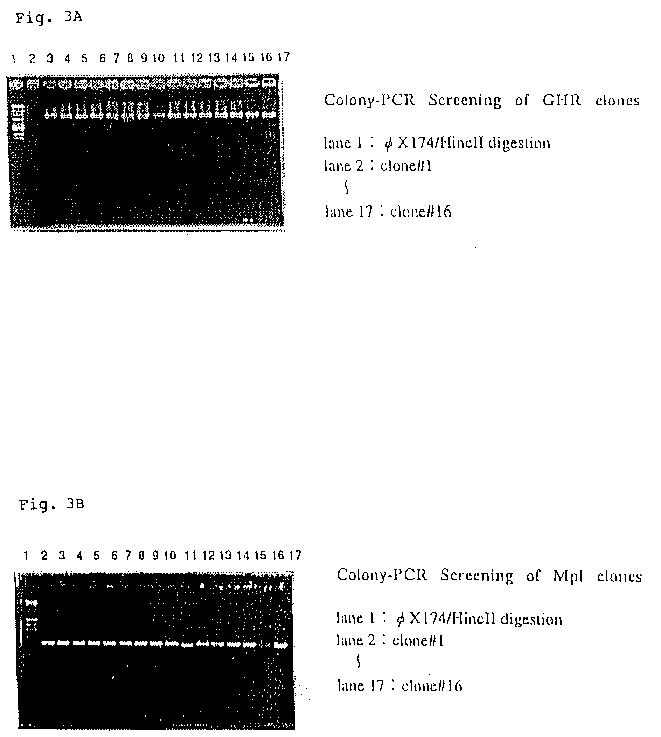

Fig. 3B depicts an electrophoretic analysis for colony-PCR screening of Mp1 clones, as conducted in Example 1 (e), in which;
   Lane 1 corresponds to λ/HindIII & φX174/HincII double digestion,
   while Lanes 2 through 17 correspond individually to Clone #1 through Clone #16, and it is added that PCR was conducted with each clone lysate using Primer T7 and GR 4 to thereby select eight clones in total of Clones #2, #3, #4, #5, #6, #7, #8 and #9 with the given DNA sequence;

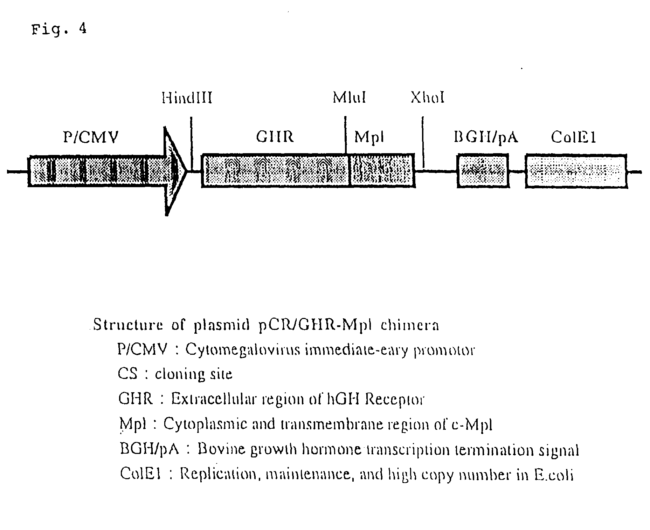

Fig. 4 illustrates the gene structure of the plasmid for expression of PCR GHR/Mp1 chimera receptor as constructed in Example 2, in which the signs have the individual following meanings:
   - P/CMV:: Cytomegalovirus immediate-early promoter
   - CS :: Cloning site
   - GHR :: Extracellular region of hGH receptor
   - Mp1 :: Cytoplasmic and transmembrane region of c-Mp1
   - BGH/pA;: Bovine growth hormone transcription termination signal
   - ColEl :: Replication, maintenance, and high copy number in E. coli;

   In order to construct the plasmid for expression of the GHR/MPl chimera receptor, there was used the recombinant plasmid generated by amplifying the extracellular-region gene of GHR and the gene ranging from the transmembrane region to the cytoplasmic region of c-Mp1 by the PCR, followed by incorporation into the TA cloning site to identify the total nucleotide sequence through DNA sequencing conducted using the primer complementary to the T7 and SP6 promoter region or the primer complementary to the nucleotide sequence of the cloned DNA. The recombinant plasmid comprises the extracellular nucleotide sequence of GHR inserted downstream of the cytomegalovirus promoter, the nucleotide sequence of the transmembrane and cytoplasmic region of c-Mp1 located downstream via the cleavage site of the restriction enzyme Mlul and the terminal signal of bovine growth hormone situated thereafter.
Fig. 5 illustrates results of the proliferative activity test of serum on BaF/GM cells as conducted in Example 4 (a), which indicate that BaF/GM cells exhibits growing activity in the presence of 10 % FBS, whereas BaF cells do not grow, but that neither BaF/GM cells nor BaF cells exhibit proliferative activity against 10 % of HS; it is also revealed that the medium containing both 10 % each of FBS and HS does not give rise to growth BaF3 cells but produces growth maintaining effect equivalent to that of BSA;
Fig. 6 is a graph showing the quantitative determination results of bGH contained in FBS by the cell proliferation analysis method using BaF/GM cells, as conducted in Example 4 (b); it is indicated that BaF/GM cells exhibit bGH-dependent proliferative activity in the detectable concentration range of 10 through 100 ng/ml, which sensitivity range is lower by one order as compared with the one for hGH, thus representing the species-specificity of the chimera receptor , the amount of bGH contained in FBS was determined by plotting against the graph as a calibration curve (216 ng/ml in PBS);
Fig. 7 is a graph showing the determination results of proliferative activity of hGH on BaF/GM cells as conducted in Example 4 (d), which indicate that BaF/GM cells exhibit hGH-dependent proliferation in the determinable concentration range of 1 through 100 ng/ml;
Fig. 8 depicts graphs showing the analysis results of responses of BaF/GM cells to hGH by a cytosenser with extracellular acidification rate, as conducted in Example 5, wherein Fig. 8A is a graph of acidification rate versus time, while Fig. 8B is a graph of acidification rate versus hGH concentration; as is evident from the graphs.
   BaF/GM cells exhibit rises in extracellular acdification rate in the hGH-concentration dependent manner, with the acidification rate showing saturation in increased hGH concentrations as high as 1 ug/ml; and
Fig. 9 is a graph showing a number of receptors bound per BaF/GM cell as analyzed and calculated in Example 6, whereby the determinable range for hGH by RRA using BaF/GM Cells is found to range 1 to 100 ng, with IC₅₀ being at 10 ng.

### Example 1: Cloning of the genes for GHR and c-Mp1

### Reagents and materials:

Human liver poly A⁺ mRNA (CLONTECH), human fetal liver poly A⁺ mRNA (CLONTECH), Kination Kit (TOYOBO), cDNA Synthesis Module (Amersham), LA Taq DNA polymerase (TAKARA), Ampli-Taq DNA polymerase (PERKIN-ELMER), Eukaryotic TA Cloning Kit (Invitrogen), T4DNA ligase (TOYOBO),GeneClean II (BIO 101), PRM Kit (BIO 101), Dye Terminator Cycle Sequencing Core Kit (PERKIN-ELMER), Gel-Mix 6 (6% Sequencing Gel Solution Ultra PURE) (GIBCO BRL), Sea Kem GTG Agarose (FMC Bio-Products), NueSieve 3:1 Agarose (FMC Bio-Products), SOC medium(GIBCO BRL), L-broth powder (TAKARA), Bacto Agar (DUFICO), 50 x TAE (Nippon Gene), Ampicillin (SIGMA), 20 mg/ml ethidium bromide solution (Nippon Gene).

### (a) Primers for PCR amplification, and primers for colony PCR and DNA sequencing:

The primers for PCR amplification as well as the primers for colony PCR and DNA sequencing were designed in the below-described regions; GR-2 and Mp1-A primers were provided with restriction enzyme M1u1 (indicated by underlining), which site served as a binding site for GHR and c-Mp1:
Primers for GHR gene cloning:
   - GR-1 primer (forward):: 5'-CTACAGGTATGGATCTCTGGCAGCT-3'
   (25 mer, 36-60)
   - GR-2 primer (reverse):: 5'-GCCATGGAAACGCGTATCTTCTTCACAT-3'
   (29mer, 816-845)
Primers for c-Mp1 gene cloning:
   - Mp1-A primer (forward):: 5'-GCCACCGAGACCACGCGTATCTCCTTGGTGACCGC
   TCTG-3' (39mer, 1456-1494)
   - MP1-B primer (reverse):: 5'-TCAAGGCTGCTGCCAATAGC-3'
   (20mer, 1899-1908)
Primers for colony PCR and DNA sequencing:
   - T7 Sequencing primer:: 5'-TAATACGACTCACTATAGGG-3'
   (20mer)
   - SP6 Sequencing primer:: 5'-GATTTAGGTGACACTACTAG-3'
   (20mer)
   - GR-4 Primer (for GHR):: 5'-ATTGCGTGGTGCTTCCCATC-3'
   (20mer, 564-583)
   - MP1-B Primer (for c-Mp1):: 5'-TCAAGGCTGCTGCCAATAGC-3'
   (20mer, 1899-1908)

In order to insert cDNA obtained by PCR amplification into plasmid pCR3-Uni in the positive direction, GR-1 and Mp1-A primer were phosphorylated at their 5' ends with use of Kination Kit.

The phosphorylation reaction of the primers was carried out by adding 40 ul in total volume of a solution mixture consisting of 10-times diluted protruding end kinase buffer (4.0 ul), 10 mM dATP (4.0 ul), 15 u/ ul T4 polynucleotide kinase (2.0 ul), 10 uM GR-1 or 10 uM Mp1-A primer (20.0 ul), sterilized double distilled water (SDDW) (10.0 ul) into an Eppendorf tube with 0.5 ml capacity, mixing the solution and allowing the reaction to proceed at 37°C for 1 hr, followed by treatment at 94°C for 5 min. to terminate the reaction.

### (b) Synthesis of cDNA from poly(A) mRNA originated from human liver and human fetal liver (Figs. 1 A and 1B):

Synthesis of cDNA from poly(A) mRNA originated from human liver and human fetal liver was conducted through the reaction under the following conditions, while using cDNA synthesis module:

A total 40 ul volume of a solution mixture consisting of first strand synthesis reaction buffer (4 ul), sodium pyrophosphate solution (1 ul), human placental ribonuclease inhibitor (1 ul), dNTP mixture (2 ul), random hexanucleotide primer (2 ul), 1 ug/ml poly(A) mRNA 91 ul), AMV reverse transcriptase (1 ul), and SDDW (8 ul) was placed into an Eppendorf tube with 0.5 ml capacity, followed by mixing, and the reaction was allowed to proceed at 42°C for 1 hr to synthesize the first strand DNA.

Then, the above reaction solution was admixed with 100 ul in total volume consisting of first strand cDNA reaction buffer (20 ul), second strand synthesis buffer (40 ul), 0.8 unit/ul ribonuclease H (1 ul), 3.5 units/ul DNA polymerase (5.6 ul) and SDDW (32.4 ul), and the reaction was allowed to proceed for 1 hr at 12°C and then at 22°C for 1 hr, followed by heating treatment for 10 min at 70°C to terminate the reaction to synthesize the second strand DNA. Furthermore, the reaction-terminated solution was admixed with 1 ul of T4 DNA polymerase (4 units/ul), and the reaction was allowed to proceed at 37°C for 10 min and then terminated by adding 2 ul of 0.5M EDTA (pH 8.0). Subsequently, the solution was subjected to the procedures of phenol extraction and ethanol precipitation in accordance with the method described in Current Protocols in Molecular Biology (2.1.1) to purify cDNA, which was then dissolved in 50 ul of TE buffer.

### (c) PCR Amplification of GHR and c-Mp1 with LA Taq DNA polymerase and extraction of DNA from agarose gel by use of Gene Clean II:

Amplification of the extracellular region of GHR:
   Primer set: Forward GR-1, Reverse GR-2
   Used cDNA : cDNA from the human liver.
The transmembrane and cytoplasmic regions of c-Mp1:
   Primer set: Forward Mp1-A, Reverse Mp1-B.
   Used cDNA : cDNA from the human fetal liver.

PCR Amplification was conducted by placing 50 ul in total volume of a solution mixture consisting of the 10-times diluted reaction solution (5.0 ul), 2.5 mM dNTP mixture (8.0 ul), 5 uM phosphorylated forward primer (4.0 ul), 10 uM reverse primer (2.0 ul), 5 units/ul LATaq DNA polymerase (0.5 ul), cDNA (5.0 ul) and SDDW (25.5 ul) into a MicroAmp reaction tube, followed by mixing, and treating the solution at 94°C for 1 min, at 60°C for 1 min and then at 72°C for 10 min to add adenine to the 3' end, whereby Gene Amp PCR System 9600 (PERKIN-ELMER) was employed as a thermal cycler.

Phenol extraction and ethanol precipitation in accordance with the procedures described in Current Protocols in Molecular Biology (2.1.1) were carried out to purify the PCR product. Thereafter, DNA was dissolved in 20 ul of TE buffer to conduct electrophoresis on a 1.0% agarose gel (Sea Kem GTG Agarose was used), and after staining with ethidium bromide, the resultant electrophoretic separation was photographed under UV irradiation, and the objective band was cut out. The DNA cut out was purified by means of Gene Clean II.

### (d) Insertion of isolated cDNAs of GHR and c-Mp1 into plasmid pCR3-Uni, followed by incorporation into E. coli TOP10F' cell line by use of the Hanahan method (Figs. 2 and 4):

Ligation was effected by placing 10 ul in total volume of the solution mixture consisting of 10-times diluted ligation buffer (1.0 ul), plasmid pCR3-Uni (2.0 ul), GHR cDNA or c-Mp1 cDNA (3.0 ul), T4 DNA ligase (1.0 ul) and SDDW (3.0 ul) into an Eppendorf tube with a 0.5-ml capacity to allow the reaction to proceed at 14°C overnight.

Transformation of E. coli was carried out by mixing gently 50 ul of E. coli TOP10F' competent cells with added cold 0.5M β-mercaptoethanol (2 ul) and furthermore adding 2 ul of the ligation product for gentle mixing, followed by standing in ice-water for 30 min; then providing thermal shock at 42°C for 30 sec to the E. coli mixed solution, which was quickly transferred into ice-water to thereby cool for 2 min; and admixing the solution with 900 ul of SOC medium warmed in advance at 37°C, followed by shake culture at 37°C for 1 hr, and sowing the culture broth on L-broth agar medium containing 50 ug/ml of ampicillin, followed by standing at 37°C overnight.

### (e) Colony-PCR screening of each expression clone to select the objective DNA of GHR from DNA of c-Mp1 (Figs. 3A, B):

Bacterial cells were taken from 16 clones with a toothpick and suspended in 10 ul of 1 % Triton x-100, and the resultant suspension was heat-treated at 100°C for 5 min to melt the bacterial cells, which was to be used as a template for PCR.

PCR Amplification was conducted by placing 20 ul in total volume of a solution mixture consisting of the 10-times diluted reaction buffer (2.0 ul), 2.5 mM dNTP mixture (2.0 ul), 10 uM T7 sequencing primer (1.0 ul), 10 uM GR-4 or 10 uM Mp1-B primer (1.0 ul), 0.5 units/ul Ampli Taq DNA polymerase (1.0 ul), E. coli lysates (2.0 ul) and SDDW (12.0 ul) into a MicroAmp reaction tube, followed by mixing, and repeating 25 times a cycle of treating the solution at 94°C for 1 min and at 55°C for 2 min, whereby Gene Amp PCR System 9600 (PERKIN-ELMER) was employed as a thermal cycler.

10 ul of the PCR product was electrophoresed on a 3 % agarose gel and after staining with ethidium bromide, the electrophoretic separation was photographed under UV irradiation to perform the clone selection.

### (f) Purification with RPM Kit and DNA sequencing with Dye Terminator Cycle Sequencing Core Kit of the plasmid from the clones found to undergo insertion to identify the total DNA sequence of GHR or c-Mp1:

Preparation of plasmid DNA was carried out by picking up a single colony of each clone, followed by sowing on a L-broth medium containing 50 ug/ml concentrated ampicillin to conduct shake culture at 37°C overnight, and recovering the bacterial cells from 1.5 ml of each culture broth to purify plasmid with use of RPM Kit; The plasmid prepared in this manner was subjected to DNA sequencing.

PCR Amplification was conducted by placing 20 ul in total volume of a solution mixture consisting of the Reaction premix (8.0 ul), 5 uM sequencing primer for T7, SP6 or Gr4 (1.0 ul), plasmid DNA (3.5 ul) and SDDW (12.0 ul) into a MicroAmp reaction tube, followed by mixing, and repeating 25 times a cycle of treating the solution at 96°C for 30 sec, at 50°C for 15 sec and at 60°C for 4 min, whereby Gene Amp PCR System 9600 (PERKIN-ELMER) was employed as a thermal cycler.

In accordance with the procedure as described in the Instructions for Use attached to the kit, ethanol precipitation was effected to purify the PCR product, and the resultant DNA was dissolved in 3 ul of loading buffer (a buffer prepared by mixing deionized formamide with 25 mM EDTA (pH 8.0) containing 50 mg/ml concentrated blue dextran at a volume ratio of 5:1), and the solution was heat-treated at 90°C for 2 min and quickly cooled with ice water to denature DNA into single-strand.

A polyacrylamide gel for DNA sequencing was prepared from Gel-Mix6 and set onto the DNA sequencer, to which the DNA sample was then applied. The miscellaneous steps were effected in accordance with the procedure described in the Instructions for Use attached to the kit.

### Example 2 Construction of GHR/Mp1 chimera gene (Figs. 4A, 4B)

### Reagents and Materials

Restriction enzyme HindIII (TAKARA), restriction enzyme Mlul (TAKARA) and restriction enzyme Xhol (TAKARA), along with other reagents being "Special-Grade" reagents as supplied by Wako Pure Chemicals.

### (a) Construction of GHR/Mp1 chimera gene with use of clone GHR-Ex #4 and Mp1-Cp#7:

Double digestion with restriction enzymes Mlul and Xhol was conducted by placing 10 ul in total volume of a solution mixture consisting of the 10-times diluted H universal buffer (1.0 ul), 10 units/ul Mlul (0.5 ul), 14 units/ul Xhol (0.5 ul), GHR-Ex#4 or Mp1-Cp#7 plasmid DNA (2 ul) and SDDW (6 ul) into an Eppendorf tube with a 0.5 ml capacity, followed by mixing, and allowing the reaction to proceed at 96°C for 4 hrs, followed by treatment at 70°C for 10 min to deactivate the restriction enzymes.

The sample prepared in this manner was applied to a 2.2 % agarose gel (Sea Kem GTG agarose gel used) to conduct electrophoresis. After staining with ethidium bromide, subsequently, the electrophoretic separation was photographed under UV irradiation, and the objective band was cut out to thereby purify DNA with Gene Clean II.

### (b) Insertion into plasmid GHR-Ex#4 of the double-digested fragment of Mp1-CP#7, followed by incorporation into E. coli TOP10F'' cell line with use of the Hanahan method:

Ligation was effected by placing 20 ul in total volume of the solution mixture consisting of 10-times diluted ligation buffer (2.0 ul), plasmid GHR-Ex#4 (2.0 ul), Mp1-Cp#7 fragment (8.0 ul), T4 DNA ligase (2.0 ul) and SDDW (6.0 ul) into an Eppendorf tube with a 0.5-ml capacity to allow the reaction to proceed at 14°C overnight.

Transformation of E. coli was carried out by mixing gently 50 ul of E. coli TOP10F' competent cells with added cold 0.5M β-mercaptoethanol (2 ul) and furthermore adding 2 ul of the ligation product for gentle mixing, followed by standing in ice-water for 30 min; then providing thermal shock at 42°C for 30 sec to the E. coli mixed solution, which was quickly transferred into ice to thereby cool for 2 min; and admixing the solution with 900 ul of SOC medium warmed in advance at 37°C, followed by shake culture at 37°C for 1 hr, and sowing the culture broth on L-broth agar medium containing 50 ug/ml of ampicillin, followed by standing at 37°C overnight for cultivation.

### (c) Colony-PCR screening to select the GHR/Mp1 gene:

Bacterial cells were taken from 16 clones with a toothpick and suspended in 10 ul of 1 % Triton X-100, and the resultant suspension was heat-treated at 100°C for 5 min to melt the bacterial cells, which was to be used as a template for PCR.

PCR Amplification was conducted by placing 20 ul in total volume of a solution mixture consisting of the 10-times diluted reaction buffer (2.0 ul), 10 uM MGR-1 primer (1.0 ul), 10 uM Mp1-primer (1.0 ul), 0.5 units/ul Ampli Taq DNA polymerase (1.0 ul), E. coli lysates (2.0 ul) and SDDW (12.0 ul) into a MicroAmp reaction tube, followed by mixing, and repeating 25 times a cycle of treating the solution at 94°C for 1 min and at 55°C for 2 min, whereby Gene Amp PCR System 9600 (PERKIN-ELMER) was employed as a thermal cycler.

10 ul of the PCR product was electrophoresed on a 10 % agarose gel (Hue Sieve 3:1 agarose used) and after staining with ethidium bromide, the electrophoretic separation was photographed under UV irradiation.

### (d) Purification of the plasmid from the clones having the chimera gene formed and confirmation of inserted DNA with restriction enzymes:

Preparation of the objective plasmid DNA was carried out by picking up a single colony of each clone, followed by sowing on a L-broth medium containing 50 ug/ml concentrated ampicillin to conduct shake culture at 37°C overnight, and recovering the bacterial cells from 1.5 ml of each culture broth to purify plasmid with use of RPM Kit; The plasmid prepared in this manner was subjected to restriction enzyme analysis and DNA sequencing.

Restriction enzyme analysis was performed by placing 10 ul in total volume of a solution mixture consisting of the 10-times diluted H universal buffer (1.0 ul), 12 units/ul HindIII (0.5 ul), 10 units/ul Mlul (0.5 ul), 14 units/ul Xhol (0.5 ul), plasmid DNA (3.0 ul) and SDDW (4.5 ul) into an Eppendorf tube with a 0.5-ml capacity, followed by mixing, and allowing the reaction to proceed at 96°C for 4 hrs, followed by treatment at 70°C for 10 min to deactivate the restriction enzymes.

10 ul of the reaction solution was electrophoresed on a 3.0 % agarose gel (Hue Sieve 3:1 agarose used) and after staining with ethidium bromide, the electrophoretic separation was photographed under UV irradiation.

### Example 3: Establishment of a hGH-dependent cell line BaF/GM

### Reagents and materials

Chloramphenicol (SIGMA), lysozyme (SIGMA), TE-saturated phenol (Nippon Gene), BaF3 (Cell Development Bank of Institute of Physical & Chemical Research),WEHI-1 (Cell Development Bank of Institute of Physical & Chemical Research), PBS (Nissui Seiyaku), RPMI 1640 medium (Nissui Seiyaku), fetal bovine serum (JRH BIOSCIENCES), 200 mM L-glutamine (GIBCO BRL), DEAE-dextran (SIGMA), gentamicin (SIGMA), geneticin (G418, GIBCO BRL), hGH (Nihon Chemical Research), along with other reagents being "Special-Grade" reagents as supplied by Wako Pure Chemicals.

### (a) Selection of clone pCR3-Uni/GM#2-15 for integration into animal cells:

Plasmid DNA was prepared in large quantities by picking up a single colony of selected clones, followed by sowing on L-broth medium containing 50-ug/ml concentrated ampicillin to conduct shake culture at 37°C overnight, then transferring the resultant culture broth into 500 ml of L-broth medium containing 500-ug/ml concentrated ampicillin to thereby conduct shake culture at 37°C until the culture broth showed 1.0 of absorbance at a wavelength of 600 nm, and adding 2.5 ml of a 34-ng/ml concentrated solution of chloramphenicol in ethanol to effect shake culture at 37°C overnight. The bacterial cells were recovered and the plasmid DNA was purified in accordance with the procedure described in Current Protocols in Molecular Biology (9.1.5).

### (b) Incorporation of plasmid pCR3-Uni/GM into BaF3 cells with use of DEAE-dextran method and selection with G 418 and hGH to establish the hGH-dependent cell line:

In order to utilize the supernatant of WEHI-3 cell culture as a source for mIL-3 preparatory to the cells usable in the transformation, the said cells were cultivated on RPMI 1640 medium (containing 10 % of bovine fetal serum and 50 ug/ml of gentamicin) to separate out a culture supernatant. The resultant supernatant was filtered through a membrane of 0.22 um in pore size (0.22 um disposable filterware: Nalgene), and the filtrate was frozen for storage. BaF3 cells were cultivated on a medium consisting of RPMI 1640 medium supplemented with 10 volume % of the supernatant of WEHI-3 culture as a source for mIL-3.

Transformation of BaF3 cells was carried out in accordance with the DEAE-dextran method; BaF3 cells during the logarithmic growth phase were sowed on a medium at a density of 5 x 10⁵ cells/ml and cultivated overnight in a CO₂ incubator. Then, Plasmid pCR3-Uni/GM sterilized through the ethanol precipitation treatment was diluted with PBS to a concentration of 10 ug/ml, and 170 ul of the resultant plasmid solution was admixed with 170 ul of a 1:1 solution mixture of a 2 mg/ml-concentrated solution of DEAE-dextran in 0.9 % NaCl with PBS.

BaF3 Cells after being cultivated overnight were washed with PBS twice, and 2 x 10⁶ cells were separated out by counting. After the supernatant was removed by centrifugation, the plasmid/DEAE dextran solution mixture was added to the cells, followed by gentle stirring and treatment at room temperature for 5 min. The cells were washed with PBS twice and cultivated on a medium for BaF3 cells in a CO₂ incubator for 48 hrs.

Selection of the transformed cells with G 418 and hGH was performed by firstly cultivating the transformant cells on a medium for BaF3 cells containing 400 ug/ml concentrated G 418 for 2 weeks, during which the medium was exchanged every several days, and then cultivating G-418 resistant cells for 1 week on a medium supplemented with 100-ng/ml concentrated hGH in place of a supernatant of wEHI-3 cell culture as a source of mIL-3 to select the cells capable of proliferating in a hGH-dependent manner, during which period the medium was exchanged every two days. The finally obtained hGH-dependent cells were considered as the BaF/GM cell (Deposition No. FERM P-16255).

### Example 4: Cell proliferation analysis with the MTT method

### (a) Assay of cell proliferative activities with use the MTT method:

It was observed that the BaF/GM cells, even after being cultivated on a medium not supplemented with hGH, can demonstrate cell proliferation without synchronizing with the G₀ phase of cell cycle. Such results are assumed to suggest that bovine growth hormone (bGH) contained in bovine fetal serum responds to GHR as expressed in the BaF/GM cell.

In light of the above, comparative investigation was conducted on different media containing fetal bovine serum (FBS), horse serum (HS) and bovine serum albumin, respectively, wherein the cell-proliferative activity was assayed by use of the cell titer 96 non-radioactive cell proliferation assay method (MTT method) (Fig. 5).

The cell proliferation analysis was effected by cultivating BaF/GM cells on RPMI 1640 medium containing 100 ng/ml of hGH overnight, with BaF3 cells as a control being cultivated on a medium for BaF3 cells overnight, then washing the cells with PBS three times, followed by further cultivation on serum-free RPMI 1640 medium for 5 hrs, diluting the cells with RPMI 1640 medium containing individually 10 % of FBS, 10 % of HS and 1 % of BSA to the cell concentration of 300,000 cells/ml, respectively, and seeding the diluted cell solutions in 100 ul portions into 96 wells for cultivation for 22 hrs., followed by determination with 3 wells by the following procedure:

After cultivation for 22 hrs, 15 ul of the dye solution was added to each well, which after cultivation for 4 hrs, was furthermore admixed with 100 ul of the solubilization/stop solution, followed by standing at room temperature overnight. Absorbance at a wavelength of 570 nm was measured for each well by a plate reader (Spectra Max 250: Molecular Devices), and each measurement data was analyzed by use of a software package for analysis (Soft Max Pro: Molecular Devices), wherein the culture broth from a 1 %-BSA containing medium was used as a blank for the absorbance measurement.

### (b) Quantitative determination of bGH contained in FBS by means of the cell proliferation analysis (the MTT method) using the BaF/GM cell (Fig. 6):

The BaF/GM cells were cultivated overnight on RPMI 1640 medium (containing 10 % of FBS) containing 100 ng/ml of hGH, and the grown cells were washed with PBS three times and cultivated for 16 hrs. on RPMI 1640 medium containing 10 % HS in place of FBS to thereby synchronize with the G₀ stage of cell cycle, followed by seeding into a 96-well flat-bottom plate for cultivation at a density of 30,000 cells/well.

bGH was distributed in 10-ul portion into wells to the different concentrations (ng/ml) of 0.39, 0.78, 1.56, 3.12, 6.25, 12.5, 25.0, 50.0, 100.0 and 200.0 to prepare standard samples, while FBS as such and after being diluted 10 times and 100 times, respectively, was also added in 10-ul portion for dilution, and all measurements were taken with every 3 wells whereby the amount of bGH contained in PBS was determined by plotting FBS values on the calibration curve.

### (c) Assay of cell proliferative activity of hGH on BaF/GM cells by means of the MTT method (Fig. 7):

The BaF/GM cells were cultivated overnight on RPMI 1460 medium (containing 10 % of FBS) containing 100-ng/ml concentrated hGH, and the grown cells were washed with PBS three times and were furthermore cultivated for 16 hrs on RPMI 1460 medium containing 10 % of HS in place of FBS to thereby synchronize with the G₀ stage of cell cycle followed by seeding into a 96-well flat-bottom plate at a density of 30,000 cells/well. hGH was distributed in 10-ul portion into wells to the different concentrations (ng/ml) of 0.39, 0.78, 1.56, 3.12, 6.25, 12.5, 25.0, 50.0, 100.0 and 200.0 . All measurements were taken with every 3 wells.

### Example 5 Cytosensor analysis of response to hGH of the BaF/GM cells through extracellular acidification rate (Fig. 8):

Synchronization of the BaF/GM cells with the G₀ stage of cell cycle was regulated by cultivating the BaF/GM cells overnight on PRMI 1460 medium (containing 10 % of FBS) containing 100 ng/ml of hGH, and recovering the grown cells, followed by washing with PBS three times and cultivation for 16 hrs on RPMI 1640 medium containing 10 % of HS.

### Cytosensor preparation and analysis:

An agarose entrapment medium was dissolved over a warm-water bath and placed into a heat block at 37°C, followed by standing. The cells were washed with a measuring medium (RPMI 1640 medium , pH 7.4, containing 4.1 % of human serum albumin) for cytosensor and adjusted to a cell density of 1.3 x 10⁷ cells/ml. The cell solution was mixed with the agarose solution at a ratio of 3 : 1, and 7 ul of the solution mixture was immediately placed in the center of a capsule cup having a spacer intervened. After the gel solidified, 2 ml of the medium was poured outside of the capsule, while 0.5 ml of the medium was added gradually to the inside of the capsule cup, and a capsule insert was placed in each capsule and sunk to the bottom quietly. The subsequent procedure was conducted by following Operation Manual for Cytosensor.

After the extracellular acidification ratio for the BaF/GM cells set into Cytosensor was stabilized, the measuring media containing individually 3.90, 15.6, 62.5, 250.0 and 1,000.0 (ng/ml) concentrated hGH were flown through the cells for 15 min, followed by switching to the measuring medium, whereby 100 ng/ml TPO was used as a control.

### Example 6 Radiorecepter analysis (Fig. 9)

### Radioreceptor analysis of hGH with use of BaF/GM cells and calculation of the dissociation constant Kd and number of receptors per cell through Scatchard analysis:

BaF/GM cells were adjusted to a density of 2.8 x 10⁷ cells/ml by cultivating the BaF/GM cells overnight on RPMI 1640 medium (10 % FBS) containing 100 ng/ml of hGH, recovering the grown cells, followed by washing with PBS three times, and further cultivating the washed cells on RPMI 1640 medium containing 10 % of HS, followed by recovery and washing with RRA medium (RPMI 1640 medium containing 0.5 % of PBS) once.

Radioreceptor analysis was carried out by preparing solutions containing different amounts of hGH per 100 ul (i.e., 2,000, 1,000, 500, 250, 125, 62.5, 31.25, 15.62, 7.81, 3.91, 1.95, 0.98, 0.49 and 0.24 ng) with use of RRA medium, then placing 100 ul each of the resultant solutions of hGH in individual Kohken tubes, adding 100 ul of a ¹²⁵I-labeled hGH solution (3.33 kBq/ml) to each tube, followed by shake culture at 4°C for 2 hrs to allow the reaction to go to conclusion, adding 2 ml of cold RRA medium to each tube, followed by stirring and centrifugation at 3,000 rpm at 4°C for 30 min, removing the supernatant and determining radioactivity per 30 sec for each tube with use of a gamma-ray counter, wherein every measured amounts of radioactivity were expressed as a mean value for two radioactivity determinations.

The resultant data were analyzed by resistance plotting and Scatchard plotting to determine IC₅₀ and dissociation constant Kd value as well as the number of receptors per cell.

Sequence Listing No. 1
Base and amino-acid sequences for GHR-Mpl chimera receptor
Length of sequence: 1224 base pairs; 408 amino acids
Topology: Linear
Type: cDNA and protein
Feature: Nucleic-acid and amino-acid sequences containing the
initiation codon and linker

17 26 35 44 53 62
5' ATG GAT CTC TGG CAG CTG CTG TTG ACC TTG GCA CTG GCA GGA TCA AGT GAT GCT
--- --- --- --- --- --- --- --- --- --- --- --- --- --- --- --- --- ---
Met Asp Leu Trp Gln Leu Leu Leu Thr Leu Ala Leu Ala Gly Ser Ser Asp Ala

71 80 89 98 107 116
TTT TCT GGA AGT GAG GCC ACA GCA GCT ATC CTT AGC AGA GCA CCC TGG AGT CTG
--- --- --- --- --- --- --- --- --- --- --- --- --- --- --- --- --- ---
Phe Ser Gly Ser Glu Ala Thr Ala Ala Ile Leu Ser Arg Ala Pro Trp Ser Leu

125 134 143 152 161 170
CAA AGT GTT AAT CCA GGC CTA AAG ACA AAT TCT TCT AAG GAG CCT AAA TTC ACC
--- --- --- --- --- --- --- --- --- --- --- --- --- --- --- --- --- ---
Gln Ser Val Asn Pro Gly Leu Lys Thr Asn Ser Ser Lys Glu Pro Lys Phe Thr

179 188 197 206 215 224
AAG TGC CGT TCA CCT GAG CGA GAG ACT TTT TCA TGC CAC TGG ACA GAT GAG GTT
--- --- --- --- --- --- --- --- --- --- --- --- --- --- --- --- --- ---
Lys Cys Arg Ser Pro Glu Arg Glu Thr Phe Ser Cys His Trp Thr Asp Glu Val

233 242 251 260 269 278
CAT CAT GGT ACA AAG AAC CTA GGA CCC ATA CAG CTG TTC TAT ACC AGA AGG AAC
--- --- --- --- --- --- --- --- --- --- --- --- --- --- --- --- --- ---
His His Gly Thr Lys Asn Leu Gly Pro Ile Gln Leu Phe Tyr Thr Arg Arg Asn

287 296 305 314 323 332
ACT CAA GAA TGG ACT CAA GAA TGG AAA GAA TGC CCT GAT TAT GTT TCT GCT GGG
--- --- --- --- --- --- --- --- --- --- --- --- --- --- --- --- --- ---
Thr Gln Glu Trp Thr Gln Glu Trp Lys Glu Cys Pro Asp Tyr Val Ser Ala Gly

341 350 359 368 377 386
GAA AAC AGC TGT TAC TTT AAT TCA TCG TTT ACC TCC ATC TGG ATA CCT TAT TGT
--- --- --- --- --- --- --- --- --- --- --- --- --- --- --- --- --- ---
Glu Asn Ser Cys Tyr Phe Asn Ser Ser Phe Thr Ser Ile Trp Ile Pro Tyr Cys

395 404 413 422 431 440
ATC AAG CTA ACT AGC AAT GGT GGT ACA GTG GAT GAA AAG TGT TTC TCT GTT GAT
--- --- --- --- --- --- --- --- --- --- --- --- --- --- --- --- ---/---
Ile Lys Leu Thr Ser Asn Gly Gly Thr Val Asp Glu Lys Cys Phe Ser Val Asp

To be continued

449 458 467 476 485 494
GAA ATA GTG CAA CCA GAT CCA CCC ATT GCC CTC AAC TGG ACT TTA CTG AAC GTC
--- --- --- --- --- --- --- --- --- --- --- --- --- --- --- --- --- ---
Glu Ile Val Gln Pro Asp Pro Pro Ile Ala Leu Asn Trp Thr Leu Leu Asn Val

503 512 521 530 539 548
AGT TTA ACT GGG ATT CAT GCA GAT ATC CAA GTG AGA TGG GAA GCA CCA CGC AAT
--- --- --- --- --- --- --- --- --- --- --- --- --- --- --- --- --- ---
Ser Leu Thr Gly Ile His Ala Asp Ile Gln Val Arg Trp Glu Ala Pro Arg Asn

557 566 575 584 593 602
GCA GAT ATT CAG AAA GGA TGG ATG GTT CTG GAG TAT GAA CTT CAA TAC AAA GAA
--- --- --- --- --- --- --- --- --- --- --- --- --- --- --- --- --- ---
Ala Asp Ile Gln Lys Gly Trp Met Val Leu Glu Tyr Glu Leu Gln Tyr Lys Glu

611 620 629 638 647 656
GTA AAT GAA ACT AAA TGG AAA ATG ATG GAC CCT ATA TTG ACA ACA TCA GTT CCA
--- --- --- --- --- --- --- --- --- --- --- --- --- --- --- --- --- ---
Val Asn Glu Thr Lys Trp Lys Met Met Asp Pro Ile Leu Thr Thr Ser Val Pro

665 674 683 692 701 710
 TG TAC TCA TTG AAA GTG GAT AAG GAA TAT GAA GTG CGT GTG AGA TCC AAA CAA
--- --- --- --- --- --- --- --- --- --- --- --- --- --- --- --- --- ---
Val Tyr Ser Leu Lys Val Asp Lys Glu Tyr Glu Val Arg Val Arg Ser Lys Gln

719 728 737 746 755 764
CGA AAC TCT GGA AAT TAT GGC GAG TTC AGT GAG GTG CTC TAT GTA ACA CTT CCT
--- --- --- --- --- --- --- --- --- --- --- --- --- --- --- --- --- ---
Arg Asn Ser Gly Asn Tyr Gly Glu Phe Ser Glu Val Leu Tyr Val Thr Leu Pro

773 782 791 800 809 818
CAG ATG AGC CAA TTT ACA TGT GAA GAA GAT ACG CGT ATC TCC TTG GTG ACC GCT
--- --- --- --- --- --- --- --- --- --- --- --- --- --- --- --- --- ---
Gln Met Ser Gln Phe Thr Cys Glu Glu Asp Thr Arg Ile Ser Leu Val Thr Ala

827 836 845 854 863 872
CTG CAT CTA GTG CTG GGC CTC AGC GCC GTC CTG GGC CTG CTG CTG CTG AGG TGG
--- --- --- --- --- --- --- --- --- --- --- --- --- --- --- --- --- ---
Leu His Leu Val Leu Gly Leu Ser Ala Val Leu Gly Leu Leu Leu Leu Arg Trp

881 890 899 908 917 926
CAG TTT CCT GCA CAC TAC AGG AGA CTG AGG CAT GCC CTG TGG CCC TCA CTT CCA
--- --- --- --- --- --- --- --- --- --- --- --- --- --- --- --- --- ---
Gln Phe Pro Ala His Tyr Arg Arg Leu Arg His Ala Leu Trp Pro Ser Leu Pro

935 944 953 962 971 980
GAC CTG CAC CGG GTC CTA GGC CAG TAC CTT AGG GAC ACT GCA GCC CTG AGC CCG
--- --- --- --- --- --- --- --- --- --- --- --- --- --- --- --- --- ---
Asp Leu His Arg Val Leu Gly Gln Tyr Leu Arg Asp Thr Ala Ala Leu Ser Pro

989 998 1007 1016 1025 1034
CCC AAG GCC ACA GTC TCA GAT ACC TGT GAA GAA GTG GAA CCC AGC CTC CTT GAA
--- --- --- --- --- --- --- --- --- --- --- --- --- --- --- --- --- ---
Pro Lys Ala Thr Val Ser Asp Thr Cys Glu Glu Val Glu Pro Ser Leu Leu Glu

To be continued

1043 1052 1061 1070 1079 1088
ATC CTC CCC AAG TCC TCA GAG AGG ACT CCT TTG CCC CTG TGT TCC TCC CAG GCC
--- --- --- --- --- --- --- --- --- --- --- --- --- --- --- --- --- ---
Ile Leu Pro Lys Ser Ser Glu Arg Thr Pro Leu Pro Leu Cys Ser Ser Gln Ala

1097 1106 1115 1124 1133 1142
CAG ATG GAC TAC CGA AGA TTG CAG CCT TCT TGC CTG GGG ACC ATG CCC CTG TCT
--- --- --- --- --- --- --- --- --- --- --- --- --- --- --- --- --- ---
Gln Met Asp Tyr Arg Arg Leu Gln Pro Ser Cys Leu Gly Thr Met Pro Leu Ser

1151 1160 1169 1178 1187 1196
GTG TGC CCA CCC ATG GCT GAG TCA GGG TCC TGC TGT ACC ACC CAC ATT GCC AAC
--- --- --- --- --- --- --- --- --- --- --- --- --- --- --- --- --- ---
Val Cys Pro Pro Met Ala Glu Ser Gly Ser Cys Cys Thr Thr His Ile Ala Asn

1205 1214 1223 1232
CAT TCC TAC CTA CCA CTA AGC TAT TGG CAG CAG CCT TGA 3'
--- --- --- --- --- --- --- --- --- --- --- --- ---
His Ser Tyr Leu Pro Leu Ser Tyr Trp Gln Gln Pro ***

## Claims

1. A chimera gene which is constructed by fusing the gene for the extracellular region of growth hormone receptor with the genes for the transmembrane and cytoplasmic regions of thrombopoietin receptor, or a protein encoded by the same.

2. A chimera gene or protein which has the sequences as given in Sequence Listing No. 1.

3. An expression plasmid for a chimera gene which is constructed by fusing the gene for the extracellular region of growth hormone receptor with the genes for the transmembrane and cytoplasmic regions of thrombopoietin receptor.

4. An expression plasmid for a chimera gene which possesses the genetic construction as illustrated in Fig. 4.

5. An animal cell which is transformed with a plasmid as claimed in claim 3.

6. A human-growth-hormone dependent cell line BaF/GM.

7. A gene for a derivative protein produced by subjecting a chimera receptor protein as claimed in claim 1 to replacement, deletion or addition for amino acid or peptide to such an extent as may retain its substantially homologous activity, and an animal cell having the same expressed therein.

8. A method for screening, by using an expression cell for a chimera receptor protein of the growth hormone, a hormone which shows affinity for such receptor, as well as its agonists and antagonists.
